(19) Europäisches Patentamt / European Patent Office / Office européen des brevets

(11) **EP 1 923 026 B1**

(12) **EUROPÄISCHE PATENTSCHRIFT**

(45) Veröffentlichungstag und Bekanntmachung des Hinweises auf die Patenterteilung:
**30.03.2011  Patentblatt 2011/13**

(51) Int Cl.:
***A61F 9/01*** *(2006.01)*

(21) Anmeldenummer: **06023151.1**

(22) Anmeldetag: **07.11.2006**

(54) **System zum Ablatieren der Hornhaut eines Auges**

System for ablating the cornea of an eye

Dispositif d'ablation de la cornee d'un oeil

(84) Benannte Vertragsstaaten:
**DE**

(43) Veröffentlichungstag der Anmeldung:
**21.05.2008  Patentblatt 2008/21**

(73) Patentinhaber: **Schwind eye-tech-solutions GmbH & Co. KG**
**63801 Kleinostheim (DE)**

(72) Erfinder:
• **Grimm, Anita**
**63928 Eichenbühl (DE)**

• **Arba-Mosquera, Samuel**
**63739 Aschaffenburg (DE)**
• **Klinner, Thomas**
**63743 Aschaffenburg (DE)**

(74) Vertreter: **Patentanwälte**
**Hofstetter, Schurack & Skora**
**Balanstrasse 57**
**81541 München (DE)**

(56) Entgegenhaltungen:
WO-A-01/89438    WO-A2-2005/063154
US-B1- 6 451 008

**Beschreibung**

Die Erfindung betrifft ein System zum Ablatieren von Hornhaut eines Auges nach dem Oberbegriff von Patentanspruch 1, wie es aus der WO 01/89438 A bekannt ist. Das Ablatieren erfolgt mit Hilfe eines Lasers, welcher Laserpulse abgibt. Eine Zieleinrichtung leitet die abgegebenen Laserpulse zu einem vorbestimmten Raumpunkt. Zur Vorbestimmung des Raumpunkts wird insbesondere ein Soll-Zielort zu jedem Laserpuls auf der Hornhaut festgelegt. Um diesen Soll-Zielort dann in einen absoluten Raumpunkt zu transformieren, bedarf es einer Information über einen Referenzpunkt auf der Hornhaut. Diese Information wird mit Hilfe eines sogenannten Eyetrackers gewonnen. Üblicherweise wird der vorbestimmte Raumpunkt, zu dem die abgegebenen Laserpulse geleitet werden, unmittelbar aus dem Soll-Zielort und einer Ausgabe des Eyetrackers betreffend die Lage des Referenzpunktes berechnet.

Problematisch an dem System des Standes der Technik ist, dass die Eyetracker keine ausreichend schnelle Messung erlauben: Zwar gibt es auch langsame Augenbewegungen, und die verschiedensten Arten von Augenbewegungen, zwischen denen unterschieden werden kann, können bei der Laserablation durch geeignete Maßnahmen unterbunden werden. Die schnellsten Augenbewegungen, die sogenannten sakkadischen Augenbewegung, bei denen das Auge schnell von einem anvisierten Zielort zu einem anderen Zielort wechselt, vollziehen sich in Zeiträumen, welche im Vergleich zu einer bei allen Eyetrackern des Standes der Technik auftretenden Verzögerungszeit zwischen dem Erfassen einer Augenstellung und dem Ausgeben von Koordinaten klein sind. Eine typische Verzögerungszeit eines Eyetrackers beträgt 6 bis 10 ms, also z.B. 8 ms, während das Auge bereits im 2-ms-Takt starke Bewegungen vollziehen kann. Bis der Eyetracker eine entsprechende Ausgabe ausgibt, kann das Auge bereits mehrfach seine Stellung gewechselt haben. Bei der Ansteuerung der Zieleinrichtung kann naturgemäß jeweils nur die aktuelle Eyetracker-Ausgabe und damit die verzögert ermittelte Stellung berücksichtigt werden. Es ist somit nicht sichergestellt, dass der Soll-Zielort für jeden Laserpuls erreicht wird. Das Problem wird durch den Trend zu hohen Pulsraten von 500 Hz oder mehr eher noch verschärft.

Die WO 01/89438 A beschreibt ein System zum Ablatieren von Hornhaut eines Auges, bei dem Soll-Zielorte für Laserpulse auf der Hornhaut eines Auges in einer Tabelle definiert werden, dann entsprechend diesen Soll-Zielorten Laserpulse abgegeben werden, und wobei eine Kamera ein Bild des Auges zeitgleich mit dem Laserpuls aufnimmt. Es werden aufgrund des Kamerabildes die Ist-Zielorte ermittelt. Bei denjenigen Soll-Zielorten, bei denen weniger Laserpulse aufgetroffen sind als vorgesehen, wird die Differenzanzahl ermittelt, und es werden weitere Laserpulse abgegeben, um diese Differenzanzahl aufzufüllen. Die Wirkungen von jenseits der Soll-Zielorten aufgetroffenen Laserpulsen werden nicht berücksichtigt, und es wird auch nicht berücksichtigt, wenn an manchen Soll-Zielorten mehr Laserpulse aufgetroffen sind als vorgesehen.

Es ist Aufgabe der Erfindung, einen Weg aufzuzeigen, wie eine höhere Übereinstimmung des tatsächlich mit Hilfe der Laserpulsabtragung erhaltenen Hornhautprofils mit einem Sollprofil erzielt werden kann.

Die Aufgabe wird durch ein System mit den Merkmalen gemäß Patentanspruch 1 gelöst.

Das erfindungsgemäße System zum Ablatieren von Hornhaut eines Auges umfasst einen Laser, der Laserpulse abgeben kann, eine Zieleinrichtung, die die Laserpulse zu einem vorgegebenen Raumpunkt leitet, eine Steuereinrichtung, die den Laser und die Zieleinrichtung ansteuert, und einen Eyetracker, der ständig oder wiederholt die Lage eines Referenzpunktes in oder auf dem Auge ermittelt und mit zeitlicher Verzögerung ein die Lage wiedergebendes Signal an die Steuereinrichtung abgibt. Erfindungsgemäß ist die Steuereinrichtung dazu ausgelegt, die Abgabe einer ersten Ausgabe von Laserpulsen an bestimmte Raumpunkte in Abhängigkeit von Soll-Zielorten der Laserpulse auf der Hornhaut und des jeweiligen aktuellen oder letzten Eyetracker-Signals zu steuern und aus den zeitlich verzögerten Signalen des Eyetrackers und den Soll-Zielorten die Ist-Zielorte der Laserpunkte auf der Hornhaut relativ zu dem Referenzpunkt zu berechnen.

Das erfindungsgemäße System ist somit in der Lage, die Ist-Zielortezu ermitteln. Dadurch sind dann auch Vergleiche mit den Soll-Zielortverteilungen möglich.

Bei dem erfindungsgemäßen System ist die Steuereinrichtung dazu ausgelegt, die Ist-Zielortverteilung und Soll-Zielortverteilung zu der ersten Anzahl von Laserpulsen zum Berechnen von Soll-Zielorten für weitere Laserpulse zu verwenden. Dies dient dazu zu versuchen, Fehler in der tatsächlich erzeugten Kontur bezüglich der Sollkontur dadurch auszugleichen, dass zielgenau Soll-Zielorte definiert werden, derart, dass für den Fall, dass die Soll-Zielorte genau ihr Ziel treffen, nun doch die Sollkontur erreicht wird.

Bei der Erfindung wird ermittelt, wie die tatsächliche Abtragung im Verhältnis zur Sollabtragung aussieht, und es wird eine weitere Anzahl von Laserpulsen festgelegt, um eine Korrektur vorzunehmen. Bei der Bestimmung der Ist-Zielorte wird hierbei davon ausgegangen, dass die Verzögerungszeit ermittelbar ist und somit rechnerisch einbezogen werden kann.

Bei der Festlegung der Soll-Zielorte für die weiteren Laserpulse kann man sich, um nicht immer neue Zielorte festlegen zu müssen, auf die Zielorte beschränken, die bereits Soll-Zielorte waren, also als Soll-Zielort festgelegt wurden. Es ist hierbei über den Stand der Technik hinaus vorgesehen, dies auch für Zielorte zu tun, die öfter getroffen wurden, als sie es ursprünglich sollten, nämlich die bei n Laserpulsen Soll-Zielorte sind und bei m>n Laserpulsen Ist-Zielorte sind. Dies lässt sich in vorteilhafter Weise insbesondere dann tun, wenn die gewünschte Kontur der Hornhaut auf diese Weise noch erzielbar ist.

Schließlich lassen sich dann auch noch zur Steigerung hiervon auch für Zielorte, die bisher nicht Soll-Zielorte waren, weitere Laserpulse festlegen. Auch dies kann da-Schließlich lassen sich dann auch noch zur Steigerung hiervon auch für Zielorte, die bisher nicht Soll-Zielorte waren, weitere Laserpulse festlegen. Auch dies kann dadurch notwendig erscheinen, dass eine vorbestimmte Kontur in der Hornhaut zu erzeugen ist.

Naturgemäß besteht natürlich bei der weiteren Anzahl von Laserpulsen wieder dasselbe Problem der Abweichung der Ist-Zielorte von den Soll-Zielorten. So ist bevorzugt vorgesehen, dass man eine abermalige Korrektur vornehmen kann. Es sollte natürlich immer die Gesamtanzahl der Laserpulse beschränkt werden, damit nicht zuviel Hornhaut abgetragen wird. Dies lässt sich dadurch erreichen, dass bei jedem Durchlauf die weitere Anzahl der Laserpulse im Vergleich zum vorigen Durchlauf kleiner wird.

Die oben erwähnte vorteilhafte Definition der Soll-Zielorte zu den weiteren Laserpulsen in Abhängigkeit von einer zu erzeugenden Kontur geht von der Erkenntnis aus, dass es diese Kontur ist, welche die Linseneigenschaften der Hornhaut bestimmt, die durch die Laserablation erst festgelegt werden sollen. Die Linseneigenschaften werden nun aber nicht durch den absoluten Abtrag von Hornhautgewebe festgelegt, sondern durch die Kontur, die sich insgesamt ergibt. Kam es zu Abweichungen zwischen Soll und Ist bei der Abtragung der Kontur im Rahmen der ersten oder vorherigen Pulsfolge, kann eine weitere Laserpulsfolge korrigierend definiert werden, damit nunmehr dieselbe Kontur erzeugt wird, wenn auch etwas tiefer in die Hornhaut eingeschrieben.

Die Kontur wird üblicherweise über die Differenz der Laserpulse zu einem Zielort im Vergleich zu den Laserpulsen zu einem benachbarten Zielort festgelegt. Es lässt sich somit definieren, dass die Zahl der Laserpulse zu zwei benachbarten Zielorten derart ergänzt wird, dass die gesamte Anzahl der Laserpulse, für die die beiden Zielorte Ist-Zielort sind, und der Laserpulse, für die die beiden Zielorte jeweils als Soll-Zielort im Rahmen der Ergänzung festgelegt werden, sich so ergibt, dass die Differenz zwischen dieser gesamten Anzahl, berechnet zum ersten benachbarten Zielort, und dieser gesamten Anzahl, berechnet zum zweiten benachbarten Zielort, genau dieselbe ist, wie die Differenz zwischen der Anzahl der zunächst festgelegten Laserpulse zum ersten benachbarten Zielort und der Anzahl der zunächst festgelegten Laserpulse zum zweiten benachbarten Zielort. Kurz gesagt wird zunächst die Kontur festgelegt, und damit auch die Differenz, und falls sich diese Differenz nicht in den Ist-Zielorten ergibt und somit die Istkontur von der Sollkontur abweicht, werden nochmals Laserpulse so definiert, dass sich insgesamt wieder die ursprünglich definierte Differenz, also somit die Sollkontur, ergibt.

Bevorzugt umfasst die Steuereinrichtung eine Recheneinrichtung, mit der Soll-Zielorte der Laserpulse aus einem Datensatz über das zu behandelnde Auge berechenbar sind. Der Datensatz kann insbesondere eine Information über die Fehlsichtigkeit des Auges, z. B. eine Dioptrinzahl des Auges beinhalten. Die Soll-Zielorte werden dann so definiert, dass sich eine bestimmte Sollkontur ergibt.

Nachfolgend werden bevorzugte Ausführungsformen der Erfindung unter Bezug auf die Zeichnung beschrieben, wobei

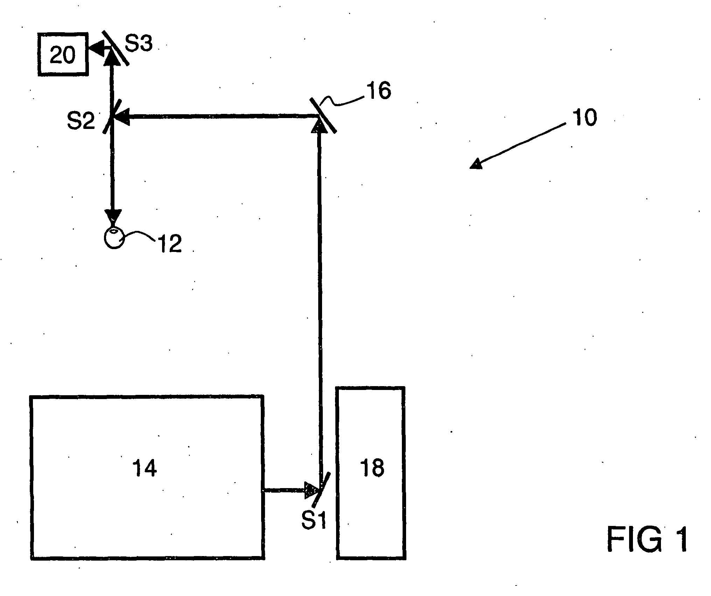

Figur 1     schematisch den Aufbau eines Systems zum Ablatieren von Horn- haut eines Auges veranschaulicht,

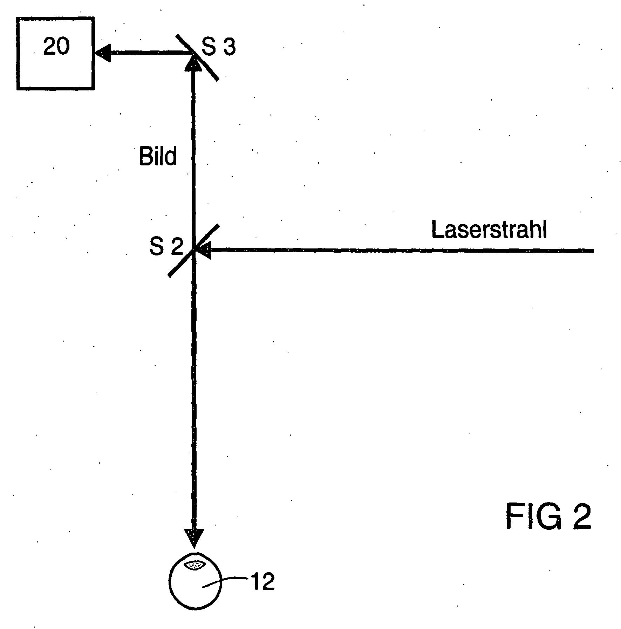

Figur 2     einen vergrößerten Ausschnitt aus Figur 1 veranschaulicht,

Figur 3     ein Koordinatensystem darstellt, mit Hilfe dessen verschiedene Ortsgrößen definiert werden,

Figur 4A    eine Sollablationsmatrix veranschaulicht,

Figur 4B    eine erste Pulsfolge entsprechend der Matrix aus 4A veranschau- licht und zugeordnet hierzu ein Beispiel für eine Ausgabe eines Eyetrackers,

Figur 5A    die Istablationsmatrix veranschaulicht, die sich aus der Pulsfolge und der Eyetracker-Ausgabe aus Figur 4B ergibt, und

Figur 5B    die Pulsfolge aus Figur 4B um die Eyetracker-Ausgabenwerte aus Figur 4B korrigiert veranschaulicht,

Figur 6B    ein erstes Beispiel für eine Folge von weiteren Laserpulsen veran- schaulicht,

Figur 7A    ein zweites Beispiel für eine Ergänzung der Ablationsmatrix aus Figur 4A, das einem Aspekt der Erfindung entspricht, veranschau- licht und

Figur 7B    ein zweites Beispiel für eine Folge von weiteren Laserpulsen ver- anschaulicht,

Figur 8A    ein drittes Beispiel für eine Ergänzung der Ablationsmatrix aus Fi- gur 4A, das einem anderen Aspekt der Erfindung entspricht, ver- anschaulicht und

Figur 8B    ein drittes Beispiel für eine Folge von weiteren Laserpulsen veran- schaulicht.

Ein im Ganzen mit 10 bezeichnetes System zum Ablatieren von Hornhaut eines Auges 12 umfasst einen Laser 14, welcher gepulste Laserstrahlung abgibt. Die Laserstrahlung von dem Laser 14 wird über ein erstes Spiegelelement S1, eine Zieleinrichtung 16 und ein zweites Spiegelelement S2 auf das Auge 12 geleitet. Die Zieleinrichtung 16 umfasst einen oder zwei verkippbare Spiegel. Die Zieleinrichtung 16 wird hierbei von einem Computer 18 als Steuereinrichtung angesteuert und jeweils so verkippt, dass ganz bestimmte Punkte auf der Hornhaut getroffen werden. Eine erste Koordinate, z. B. die x-Koordinate verläuft hierbei senkrecht zu dem auf dem Auge 12 auftreffenden Pfeil in der Bildebene, und eine zweite Koordinate, z.B. die y-Koordinate verläuft senkrecht zu dem auf dem Auge auftreffenden Pfeil 12 senkrecht zur Bildebene.

Mit Hilfe eines Eyetrackers 20 werden Bewegungen des Auges 12 verfolgt. Über das halbdurchlässige Spiegelelement S2 und einen Spiegel S3 erhält der Eyetracker ein Bild des Auges 12 und wertet dieses aus. Der Eyetracker ist insbesondere in der Lage, die Pupille des Auges 12 zu erkennen und als Referenzpunkt den Mittelpunkt der Pupildie Pupille des Auges 12 zu erkennen und als Referenzpunkt den Mittelpunkt der Pupille zu erkennen und eine Koordinate auszugeben, welche die relative Lage des Pupillenzentrums angibt.

Im Rahmen der Laserablation wird in die Hornhaut des Auges 12 durch Abtragung von Hornhautmaterial ein Profil eingeschrieben, welches wie die Oberfläche einer Linse wirkt und einen Augenfehler korrigiert. Aus der Verteilung der Laserpulse des Lasers 14 auf verschiedene Stellen auf der Hornhaut soll sich das Profil ergeben. Es werden somit zu Laserpulsen einer Laserpulsfolge Soll-Zielorte festgelegt. Dieser Soll-Zielort wird relativ zu dem Pupillenzentrum definiert, und bei jedem Laserpuls des Lasers 14 wird die Zieleinrichtung 16 so angesteuert, dass soweit als möglich der Soll-Zielort auf dem Auge tatsächlich erreicht wird. Hierzu werden die Informationen des Eyetrackers 20 genutzt: Die jeweils letzte bzw. aktuelle Information über die Lage des Pupillenzentrums und die Koordinaten des Soll-Zielorts werden dazu verwendet, den Verkippungswinkel für die Zieleinrichtung 16 festzulegen und den Laserstrahl zielgenau anzusteuern.

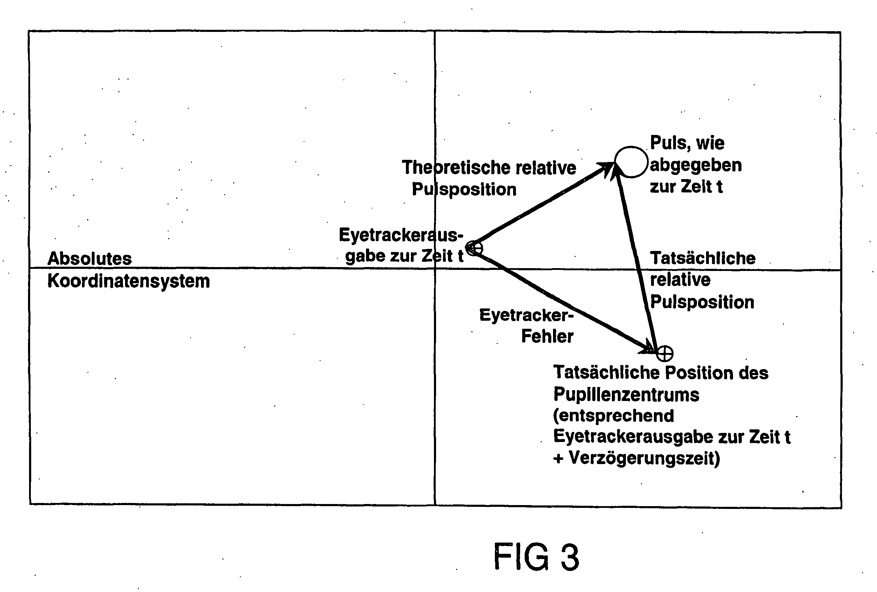

Dies wird nachfolgend anhand von Figur 3 erläutert: Figur 3 zeigt ein absolutes Koordinatensystem, das von dem Auge 12 unabhängig sei und von der Zieleinrichtung 16 verwendet wird.

Für einen bestimmten Laserpuls sei nun ein Soll-Zielort festgelegt. Der Soll-Zielort ist als Vektorpfeil festgelegt, und zwar bezogen auf das Pupillenzentrum. Dieser Vektorpfeil ist in Figur 3 mit "Theoretische relative Pulsposition" bezeichnet. Vorliegend setzt dieser Vektorpfeil nicht im Nullpunkt an, sondern in einem Punkt, der mit "Eyetracker-Ausgabe zur Zeit t" bezeichnet ist. Die Koordinaten dieses Punktes werden dementsprechend von dem Eyetracker zur Zeit t ausgegeben. Für die Abgabe des Laserpulses wird also davon ausgegangen, das dies die Koordinaten des Pupillenzentrums sind, so dass der genannte Vektorpfeil " Theoretische relative Pulsposition" zu den Koordinaten des Punktes hinzuaddiert wird. Hierbei ergibt sich ein weiterer Punkt, der mit " Puls, wie abgegeben zur Zeit t" bezeichnet ist. Dieser Punkt wird nämlich dazu verwendet, die Zieleinrichtung 16 einzustellen, die den absoluten Raumpunkt festlegt, auf den der Laser hinzielt.

Ein Problem besteht nun darin, dass der Eyetracker die Messwerte mit einer Verzögerungszeit ausgibt. Der Punkt, an dem der Vektorpfeil "Theoretische relative Pulsposition" ansetzt, zeigt somit nicht die tatsächliche Position des Pupillenzentrums zur Zeit t, sondern zur Zeit (t - Verzögerungszeit). Der Puls wurde aber zur Zeit t entsprechend dem so beschrifteten Punkt abgegeben. Um zu wissen, welcher Ist-Zielort auf der Hornhaut von dem Laserpuls getroffen wurde, muss man die tatsächliche Position des Pupillenzentrums zur Zeit t kennen, und somit die Eyetracker-Ausgabe zum Zeitpunkt t zuzüglich der Verzögerungszeit abwarten. Im Beispiel aus Figur 3 ergibt sich dann der Punkt "Tatsächliche Position des Pupillenzentrums", der entsprechend dem mit "Eyetracker-Fehler" bezeichneten Vektorpfeil von dem für die Berechnung des tatsächlich abgegebenen Pulses verwendeten Punkt abweicht. Die tatsächliche relative Pulsposition ist dann durch den Vektorpfeil, der entsprechend beschriftet ist, angegeben. Sie weicht von der theoretisch relativen Pulsposition stark ab.

Die Größen aus Figur 3 werden nun anhand konkreter Zahlenbeispiele erläutert.

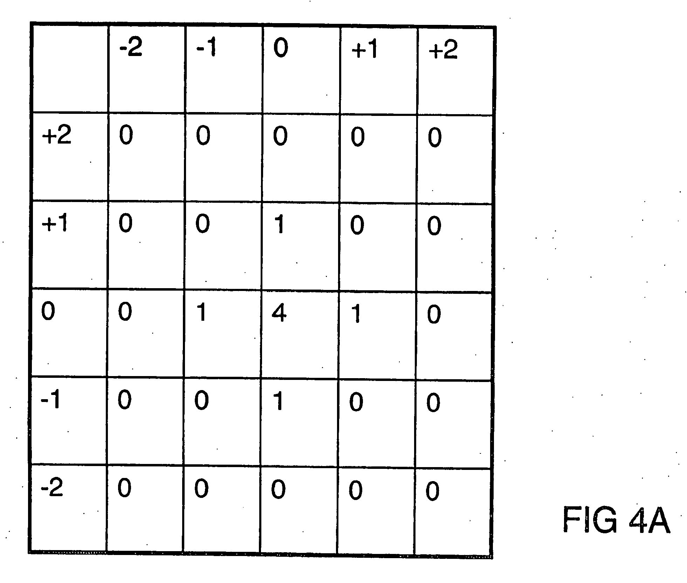

Figur 4A zeigt ein Beispiel einer vereinfachten Soll-Ablationsmatrix. Eine Ablationsmatrix legt zu bestimmten diskretisierten Koordinaten jeweils die Zahl der Laserpulse fest. Üblicherweise hat eine solche Ablationsmatrix 30 bis 1000 Zeilen und Spalten und entsprechend viele Einträge, abgegeben werden 20 000 Laserpulse (oder auch deutlich mehr). Im Beispiel von Figur 4A ist lediglich eine 5x5 Matrix dargestellt für insgesamt acht Laserpulse.

Während der Eintrag in der Ablationsmatrix aus Figur 4A für die meisten Soll-Zielorte Null ist, ist er beim Koordinatenpunkt (0, 0) = 4, beim Koordinatenpunkt (0, +1) = 1, beim Koordinatenpunkt (0, -1) = 1, beim Koordinatenpunkt (-1, 0) = 1 und beim Koordinatenpunkt (+1, 0) = 1. Wir haben es hierbei mit dem Fall zu tun, dass im Zentrum besonders viel ablatiert wird. Dies ist typischerweise der Fall, wenn der Effekt einer Konkavlinse erzielt werden soll, also Kurzsichtigkeit korrigiert werden soll.

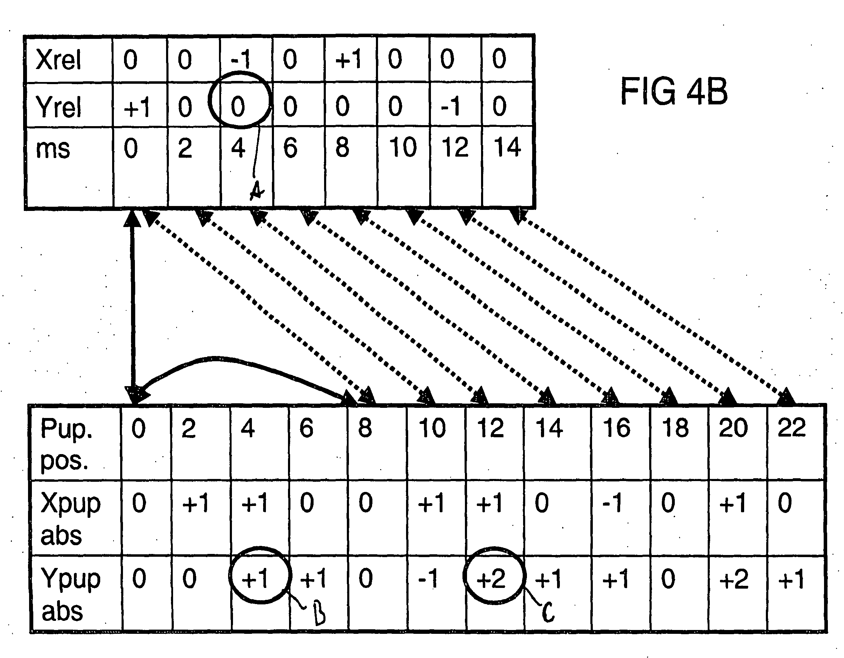

In Figur 4B ist nun eine Folge von Pulsen im 2-ms-Takt gezeigt, die anhand der Ablationsmatrix aus Figur 4A definiert wurden, wobei die Reihenfolge der Pulse hierbei mehr oder wenig willkürlich gewählt ist. Die Einträge Xrel und Yrel sind hierbei die Koordinaten aus der Ablationsmatrix, und diese sind nichts anderes als die Koordinaten des Vektors aus Figur 3, der mit "Theoretische relative Pulsposition" beschriftet ist. Im unteren Teil von Figur 4B ist nun ein Beispiel einer möglichen Eyetracker-Ausgabe zu verschiedenen Zeiten gezeigt, welche die Koordinaten Xpupabs und Ypupabs angibt, also die absoluten Stellungen der Pupillenposition. In Figur 4B ist die Situation idealisiert gezeigt, und zwar werden die Pulse synchronisiert mit den Messungen des Eyetrackers abgegeben. Der Punkt "Puls, wie abgegeben zur Zeit t" aus Figur 3 ergibt sich aus den unteren Koordinaten Xpupabs und Ypupabs zu einem bestimmten Zeitpunkt (0, 2, 4, ... in ms) durch Addition mit den entsprechenden Koordinaten Xrel und Yrel zum selben Zeitpunkt.

Es wird hierbei davon ausgegangen, dass die Zeit, die zum Berechnen dieser Summe notwendig ist, und die Zeit, die zum Steuern der Zieleinrichtung verstreicht, verschwindend gering ist im Vergleich zur Taktung von 2ms. Die in Figur 4B in idealisierender Weise gezeigte Synchronisierung könnte natürlich auch in der Realität umgesetzt werden, wenn der Eyetracker die Datenwerte nicht kontinuierlich oder in noch kleineren Zeitschritten ausgibt.

Nun ist es so, dass der Eyetracker erst mit 8 ms Verzögerung (vgl. Pfeil zwischen "0" und "8" in Figur 4B unten) die Werte Xpupabs und Ypupabs ausgibt. Während dieser 8 ms kann sich das Auge natürlich weiter bewegen, was hier in Figur 4B durch Änderungen der Koordinatenwerte im 2ms-Abstand in möglicherweise übertriebener Weise dargestellt ist.

Erst nach 8ms kann somit die tatsächliche relative Pulsposition ermittelt werden.

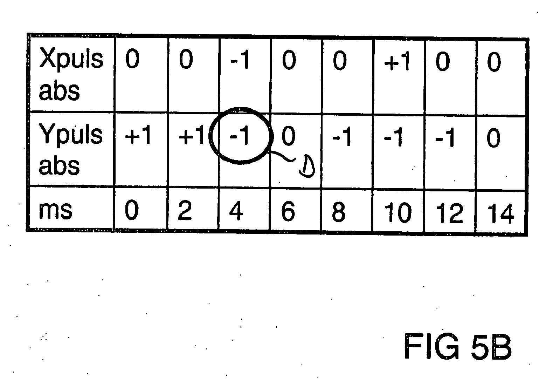

Diese Ist-Werte sind in Figur 5B zu den Zeitpunkten der Laserpulsabgabe dargestellt. Die Berechnung dieser Istwerte sei nun anhand der Y-Koordinate für den nach 4 ms abgegebenen Puls erläutert. Der Ist-Wert beträgt -1 und ist in Figur 5B mit D bezeichnet. Der Sollwert sollte 0 sein und ist in Figur 4B mit A bezeichnet. Bei der Erzeugung des Istwertes war von der Pupillenposition ausgegangen, wie ihn der Eyetracker zum Zeitpunkt 4ms ausgab, nämlich +1 (mit B bezeichnet). Die tatsächliche Pupillenposition ist erst 8ms später, zum Zeitpunkt 12ms, ermittelbar und beträgt +2. Die Pupille ist somit in Realität um einen Wert von 1 in der Y-Koordinate beim Abgeben des Laserpulses höher gewesen als vorausgesetzt. Dadurch ist der Ist-Zielort um einen Koordinatenpunkt tiefer, und liegt somit auf -1 (D) im Vergleich zur 0 (A).

In einer Formel ausgedrückt lässt sich dies wiedergeben durch:

$$A + (B - C) = D.$$

Sämtliche Istwerte aus Figur 5B lassen sich aus den Sollwerten aus Figur 4B oben zusammen mit den Pupillenmesswerten aus Figur 4B unten ermitteln, wobei sowohl der bei der Abgabe des Laserpulses berücksichtigte Wert der Eyetracker-Ausgabe als auch der 8ms später gemessene tatsächliche Wert zu berücksichtigen sind.

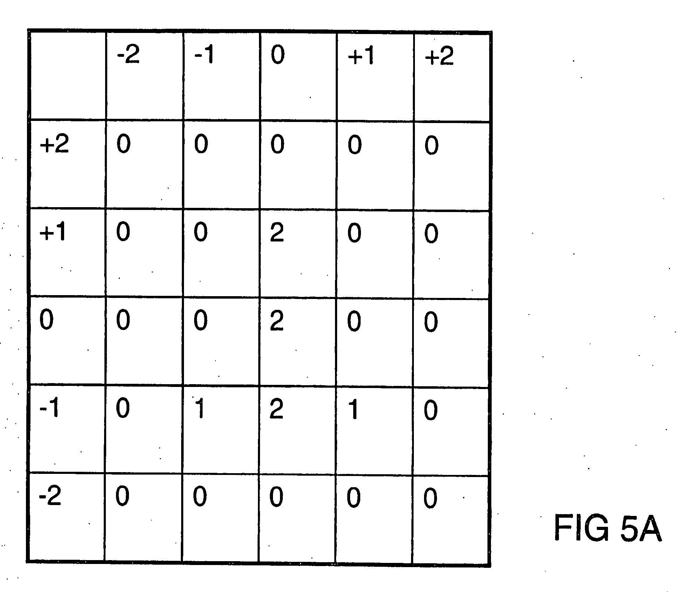

Aus der Folge der Laserpulse in Figur 5B lässt sich die Istablationsmatrix aus Figur 5A ableiten. Vergleicht man nun Ist und Soll, also Figur 5A mit Figur 4A, so wird deutlich, wie stark die gewünschte Kontur verfehlt wurde.

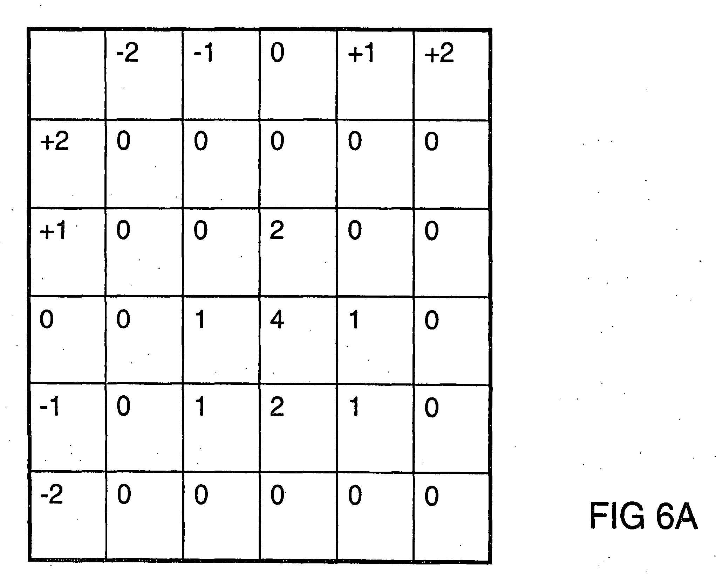

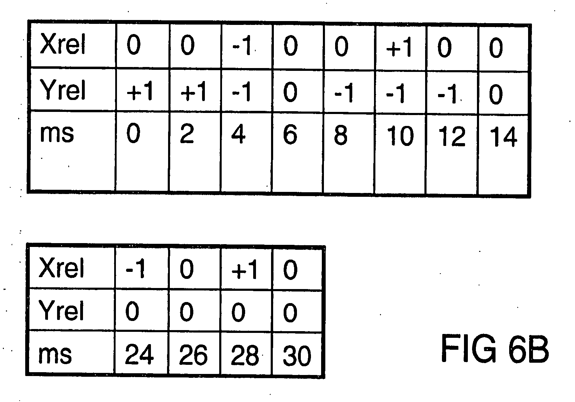

Nun ist der Weg für eine Korrektur offen. Weil keine Impulse zurückgenommen werden können - es kann ja auch kein Hornhautmaterial wiederhergestellt werden - bleibt zur Korrektur lediglich die Möglichkeit, weitere Laserpulse zu definieren. Hierfür gibt es verschiedene Möglichkeiten, die vorliegend anhand der Figuren 6, 7 und 8 beschrieben werden. Allen Möglichkeiten ist gemeinsam, dass eine neue Sollablationsmatrix erzeugt wird, und zwar so, dass die Istablationsmatrix aus Figur 5A um zusätzliche Laserschüsse ergänzt wird. Die neue Sollablationsmatrix soll hierbei die alte Sollablationsmatrix aus Figur 4A ersetzen und dementsprechend der gewünschten Hornhautkontur möglichst nahe kommen, andererseits aber nicht unberücksichtigt lassen, was im Rahmen der ersten Laserpulsfolge schon geschehen ist. Mit anderen Worten hat keine der Sollablationsmatrizen einen Eintrag, bei dem an gleicher Stelle in der Istablationmatrix aus Figur 5A der Eintrag größer wäre: Im Vergleich zu Figur 5A wird kein Eintrag weggenommen, sondern höchstens ein Eintrag hinzugefügt.

Man vergleiche nun das erste Beispiel, das in Figur 6A gezeigt ist, mit der Istablationsmatrix aus Figur 5A: Ergänzt wurden zwei Schüsse mit den Koordinaten (0, 0) und jeweils ein Schuss mit den Koordinaten (-1, 0) und ein Schuss mit den Koordinaten (+1, 0). In der neuen Sollablationsmatrix wird in der Y-Koordinate 0 die Sollablationsmatrix aus Figur 4A wiederhergestellt. Die Ausführungsform gemäß Figur 6A beinhaltet, dass nur für solche Zielorte, die weniger häufig Ist-Zielort sind als Soll-Zielort (bei denen also der Eintrag zu der entsprechenden Koordinatenkombination in der Matrix aus Figur 5A kleiner ist als in der Matrix aus Figur 4A) Laserpulse ergänzt werden. Es wird somit ergänzt, was im Vergleich zur Ursprungsmatrix noch fehlt, während nicht notwendigerweise die Fehlschüsse ausgeglichen werden. Entsprechend ergibt sich eine im unteren Bild von Figur 6B gezeigte Folge aus zusätzlichen Pulsen, die hier als mit der Zeit 24ms beginnend gezeigt sind. Dies ist bei einer Laserfrequenz von 500 Hz die früheste Zeit, bei der alle Ist-Werte berücksichtigt werden können. In der ersten in Figur 6B nochmals gezeigten Laserpulsfolge wurde der letzte Laser ja zum Zeitpunkt 14ms abgegeben, und die Verzögerungszeit von 8ms, die der Eyetracker hat, bewirkt, dass erst bei 22ms die tatsächliche Position des Pupillenzentrums ermittelt wird.

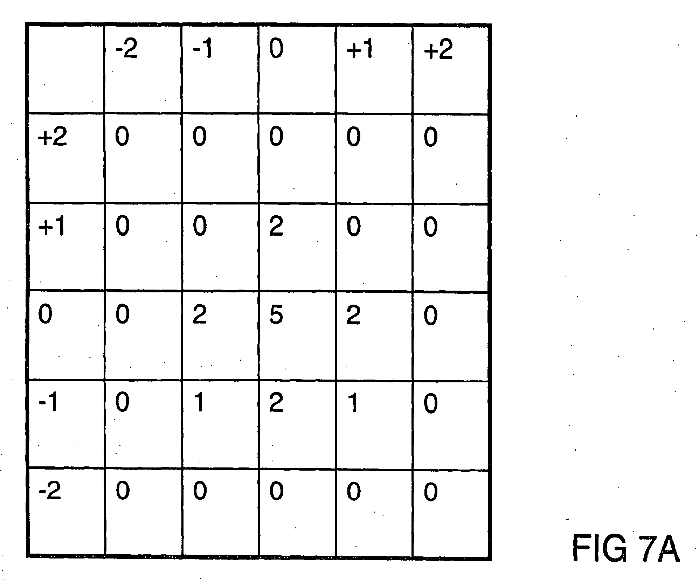

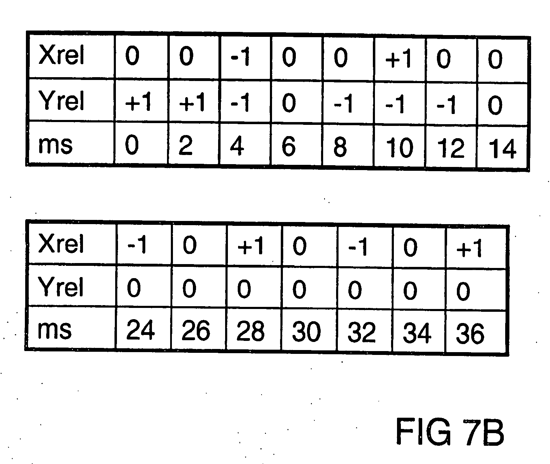

Die vorliegend nicht beanspruchte Ausführungsform gemäß Figur 6A, bei der nur ergänzt wurde, wo im Vergleich zur Ursprungsablationsmatrix Lücken entstanden sind, kann erfindungsgemäß dahingehend weitergeführt werden, dass grundsätzlich bei allen Soll-Zielorten, die überhaupt einen Eintrag in der Ursprungsablationsmatrix hatten, auch ein zusätzlicher Laserpuls definiert werden kann, insbesondere über die ursprüngliche Zahl von Laserpulsen hinaus, während bei Orten, die nicht Soll-Zielort in der ursprünglichen Ablationsmatrix waren, keine zusätzlichen Laserpulse definiert sein sollen. Entsprechend Figur 7A werden gleich sieben zusätzliche Laserpulse definiert, welche in Figur 7B auch im Einzelnen dargestellt sind. Während es bei der Ausführungsform gemäß Figur 6 darum ging, die Gesamtzahl der Laserpulse zu den Soll-Zielorten nicht zu erhöhen, steht bei Figur 7 die Gesamtkontur im Zentrum des Interesses. Die Gesamtkontur soll ja einen Linseneffekt ergeben, und bei der ursprünglichen Ablationsmatrix aus Figur 4A unterschied sich die Abtragung am Zielort (0, 0) um genau drei Punkte zu den jeweiligen Nachbarn. Diese Differenz zwischen den Laserpulsen, die zu benachbarten Zielorten definiert sind, wird nun anhand von Figur 7A wiederhergestellt. Geht man davon aus, dass die hinzugekommenen Laserpulse ihr Ziel treffen, kann man die Linsenkontur doch noch herstellen, die mit Hilfe der Ablationsmatrix aus Figur 4A hergestellt werden sollte, wenn man die beiden Fehlschüsse auf die Zielorte (-1, -1) und (+1, -1) vernachlässigt.

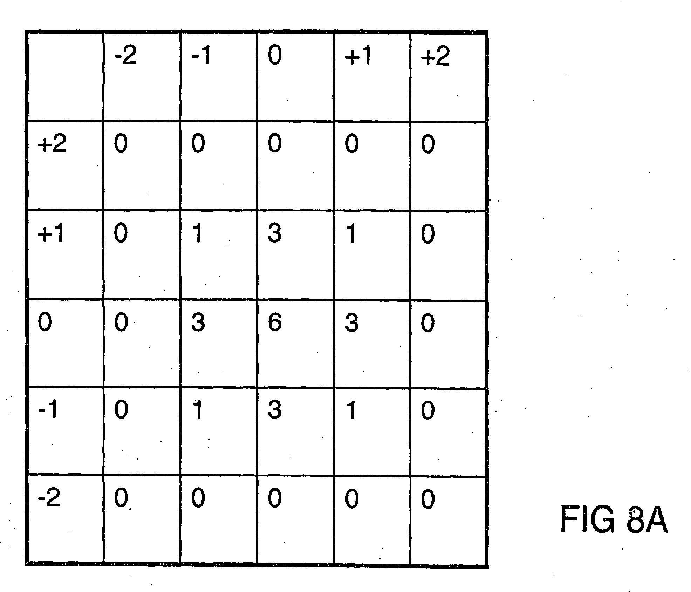

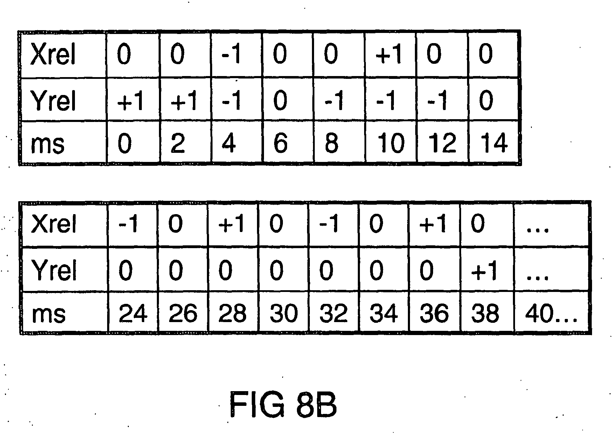

Während in Figur 7 nur zusätzliche Laserpulse zu Zielorten definiert wurden, die bereits Soll-Zielort in der ursprünglichen Matrix aus Figur 4A waren, kann zur weiteren Steigerung entsprechend Figur 8A vorgesehen sein, auch an Zielorten, die nicht Soll-Zielort waren, zusätzliche Laserpulse zu definieren. Wie oben bereits besprochen, ergibt sich die Ablationsmatrix aus Figur 8A aus der Istmatrix aus Figur 5A durch Hinzufügen weiterer Laserpulse, die in Figur 8B nur teilweise dargestellt sind. Die Hinzufügung orientiert sich hierbei an dem durch die Ursprungsablationsmatrix in Figur 4A vorgegebenen Muster. Im Unterschied zu der Ausführungsform aus Figur 7A wird die Zahl der Laserpulse weiter erhöht, damit die Kontur aus der Ursprungsablationsmatrix in Figur 4A noch genauer getroffen werden kann. Durch die weitere Erhöhung der Laserpulse im Zentrum (Zielort (0, 0)) und an den vier weiteren ursprünglichen Zielorten (-1, 0) etc. kann die Wirkung der Fehlschüsse auf die Zielorte (-1, -1) und (+1, -1) weiter ausgeglichen werden. Ein zusätzlicher Ausgleich geschieht durch zusätzliche Laserpulse mit dem Zielort (-1, +1) und (+1, +1).

Anhand der Figuren 6, 7 und 8 wurde jeweils erläutert, wie eine weitere Folge von Laserpulsen zusätzlich zu der ersten Folge von Laserpulsen (0 bis 14ms) definierbar ist. Bei der hohen Anzahl von 20 000 Laserpulsen, die bei einer herkömmlichen Laserablation abgegeben werden, ist es naturgemäß auch möglich, die Addition zusätzlicher Laserpulse mehrfach zu wiederholen. Insbesondere kann durch eine weitere Iteration jeweils das Abweichen des Ist-Zielortes der Laserpulse vom Soll-Zielort bei der vorherigen Iteration korrigiert werden. Die Zahl der hinzugefügten Laserpulse kann von Iteration zu Iteration kleiner werden. Auch der jeweils verfolgte Ansatz, welche Zielorte ausgesucht werden, kann variieren. So kann beispielsweise mit dem Ansatz gemäß Figur 8 begonnen werden, eher mehr als weniger Laserpulse hinzuzufügen, um die gewünschte Kontur zu erhalten, und bei weiteren Iterationen kann dann zu den Ansätzen übergegangen werden, wie sie anhand von Figur 7 oder Figur 6 erläutert wurden, damit die Gesamtmenge an abgetragenem Hornhautmaterial nicht übermäßig steigt.

## Patentansprüche

**1.** System (10) zum Ablatieren von Hornhaut eines Auges (12), mit einem Laser (14), der Laserpulse abgibt, mit:

- einer Zieleinrichtung (16), die die Laserpulse zu einem vorgebbaren Raumpunkt leitet,
- einer Steuereinrichtung (18), die den Laser (14) und die Zieleinrichtung (16) ansteuert,
- einem Eyetracker (20), der ständig oder wiederholt die Lage eines Referenzpunktes in oder auf dem Auge (12) ermittelt und mit zeitlicher Verzögerung ein die Lage wiedergebendes Signal an die Steuereinrichtung (18) abgibt,

wobei die Steuereinrichtung (18) dazu ausgelegt ist, die Abgabe einer ersten Anzahl von Laserpulsen an bestimmte Raumpunkte in Abhängigkeit von Soll-Zielorten der Laserpulse auf der Hornhaut und des jeweils aktuellen oder letzten Eyetracker-Signals zu steuern und aus einem jeweils zeitlich verzögert erfassten Signal des Eyetrackers (20) und den Soll-Zielorten die Ist-Zielorte der Laserpulse auf der Hornhaut relativ zu dem Referenzpunkt zu berechnen und ferner dazu ausgelegt ist, die Ist-Zielortverteilung und die Soll-Zielortverteilung zu der ersten Anzahl von Laserpulsen zum Berechnen von Soll-Zielorten für weitere Laserpulse zu verwenden,
**dadurch gekennzeichnet, dass**
entweder: nur für Zielorte, die bereits Soll-Zielorte waren, weitere Laserpulse festgelegt werden, wobei auch für Zielorte, die bei n Laserpulsen Soll-Zielorte sind und bei m>n Laserpulsen Ist-Zielorte sind, weitere Laserpulse festgelegt werden,
oder: auch für Zielorte, die bisher nicht Soll-Zielorte waren, weitere Laserpulse festgelegt werden.

**2.** System (10) nach Anspruch 1,
**dadurch gekennzeichnet,**
**dass** die Steuereinrichtung (18) eine Recheneinrichtung umfasst, mit der Soll-Zielorte der Laserpulse aus einem Datensatz über das zu behandelnde Auge berechenbar sind.

**3.** System nach Anspruch 1 oder 2,
**dadurch gekennzeichnet,**
**dass** die Zahl der Laserpulse zu zwei benachbarten Zielorten derart ergänzt wird, dass die gesamte Anzahl der Laserpulse, für die die beiden Zielorte jeweils Ist-Zielort sind, und der Laserpulse, für die die beiden Zielorte für weitere Laserpulse jeweils als Soll-Zielort festgelegt werden, sich so ergibt, dass die Differenz zwischen dieser gesamten Anzahl zum ersten benachbarten Zielort und der gesamten Anzahl zum zweiten benachbarten Zielort genau dieselbe ist wie die Differenz zwischen der Anzahl der bei der ersten Festlegung der Soll-Zielorte festgelegten Laserpulsen zum ersten benachbarten Zielort und der Anzahl der bei der ersten Festlegung der Soll-Zielorte festgelegten Laserpulse zum zweiten benachbarten Zielort.

**Claims**

1. System (10) for ablating cornea of an eye (12) with a laser (14) emitting laser pulses, including:

   - an aiming device (16) directing the laser pulses to a presettable spatial point,
   - a controller (18) driving the laser (14) and the aiming device (16),
   - an eye tracker (20) continuously or repeatedly determining the position of a reference point in or on the eye (12) and delivering a signal representing the position to the controller (18) with time lag,

   wherein the controller (18) is adapted to control the emission of a first number of laser pulses to certain spatial points depending on set target locations of the laser pulses on the cornea and of the respectively current or last eye tracker signal and to calculate the actual target locations of the laser pulses on the cornea relative to the reference point from a signal of the eye tracker (20) respectively acquired with time lag and the set target locations, and further is adapted to use the actual target location distribution and the set target location distribution to the first number of laser pulses for calculating set target locations for further laser pulses,
   **characterized in that**
   either: further laser pulses are specified only for target locations, which have already been set target locations, wherein further laser pulses are specified also for target locations, which are set target locations for n laser pulses and are actual target locations for m>n laser pulses,
   or: further laser pulses are specified also for target locations, which have not been set target locations heretofore.

2. System (10) according to claim 1,
   **characterized in that**
   the controller (18) includes a computing means, by which set target locations of the laser pulses can be computed from a data record on the eye to be treated.

3. System according to claim 1 or 2,
   **characterized in that**
   the number of the laser pulses to two adjacent target locations is supplemented such that the total number of the laser pulses, for which the two target locations are each actual target location, and of the laser pulses, for which the two target locations are each specified as a set target location for further laser pulses, results such that the difference between this total number to the first adjacent target location and the total number to the second adjacent target location is exactly the same as the difference between the number of the laser pulses specified in the first specification of the set target locations to the first adjacent target location and the number of the laser pulses specified in the first specification of the set target locations to the second adjacent target location.

**Revendications**

1. Système (10), destiné à l'ablation de la cornée d'un oeil (12), avec un laser (14), qui délivre des impulsions laser, avec :

   - un dispositif de visée (16), qui dirige les impulsions laser vers un point dans l'espace pouvant être prédéterminé,
   - un dispositif de commande (18), qui commande le laser (14) et le dispositif de visée (16),
   - un dispositif de pistage de l'oeil (20), qui recherche en permanence ou de manière répétée la position d'un point de référence dans ou sur l'oeil (12) et qui délivre, avec une temporisation, un signal, restituant la position, au dispositif de commande (18),

   moyennant quoi le dispositif de commande (18) est conçu à cet effet, pour commander la délivrance d'un premier nombre d'impulsions laser en des points précis dans l'espace en fonction de lieux de destination théoriques des impulsions laser sur la cornée et du signal du dispositif de pistage de l'oeil respectivement actuel ou dernier et pour calculer, à partir d'un signal saisi de manière respectivement temporisée du dispositif de pistage de l'oeil (20) et des lieux de destination théoriques, les lieux de destination réels des impulsions laser sur la cornée par rapport au point de référence et est conçu en outre à cet effet pour utiliser la répartition des lieux de destination réels et la répartition des lieux de destination théoriques, relatives au premier nombre d'impulsions laser, en vue du calcul de lieux de destination théoriques pour d'autres impulsions laser,
   **caractérisé en ce que**
   soit : uniquement pour les lieux de destination, qui étaient déjà des lieux de destination théoriques, d'autres impulsions laser sont définies, moyennant quoi d'autres impulsions laser sont définies même pour les lieux de destination, qui sont, dans le cas d'impulsions laser n, des lieux de destination théoriques et, dans le cas d'impulsions laser m > n, des lieux de destination réels

soit : d'autres impulsions laser sont définies même pour les lieux de destination, qui n'étaient pas des lieux de destination théoriques jusqu'à présent.

2. Système (10) suivant la revendication 1, **caractérisé en ce**
que le dispositif de commande (18) comprend un dispositif de calcul, avec lequel les lieux de destination théoriques des impulsions laser peuvent être calculés à partir d'un jeu de données sur l'oeil à traiter.

3. Système (10) suivant la revendication 1 ou 2, **caractérisé en ce**
que le nombre des impulsions laser, relatif à deux lieux de destination voisins, est complété de telle sorte que le nombre total des impulsions laser, pour lesquelles les deux lieux de destination sont respectivement un lieu de destination réel et des impulsions laser, pour lesquelles les deux lieux de destination pour d'autres impulsions laser sont respectivement définis comme un lieu de destination théorique, qu'il résulte que la différence entre ce nombre total, relatif au premier lieu de destination voisin et le nombre total, relatif au deuxième lieu de destination voisin, est exactement identique à la différence entre le nombre des impulsions laser, relatives au premier lieu de destination voisin et déterminées lors de la première fixation des lieux de destination théoriques et le nombre des impulsions laser, relatives au deuxième lieu de destination voisin et déterminées lors de la première fixation des lieux de destination théoriques.

EP 1 923 026 B1

S3

20

S2

16

10

12

14

S1

18

FIG 1

EP 1 923 026 B1

20

S 3

Bild

S 2

Laserstrahl

12

FIG 2

Puls, wie abgegeben zur Zeit t

Theoretische relative Pulsposition

Eyetrackeraus-gabe zur Zeit t

Absolutes Koordinatensystem

Tatsächliche relative Pulsposition

Eyetracker-Fehler

Tatsächliche Position des Pupillenzentrums (entsprechend Eyetrackerausgabe zur Zeit t + Verzögerungszeit)

## FIG 3

EP 1 923 026 B1

|    | -2 | -1 | 0 | +1 | +2 |
|----|----|----|---|----|----|
| +2 | 0  | 0  | 0 | 0  | 0  |
| +1 | 0  | 0  | 1 | 0  | 0  |
| 0  | 0  | 1  | 4 | 1  | 0  |
| -1 | 0  | 0  | 1 | 0  | 0  |
| -2 | 0  | 0  | 0 | 0  | 0  |

FIG 4A

EP 1 923 026 B1

FIG 4B

| Xrel | 0 | 0 | -1 | 0 | +1 | 0 | 0 | 0 |
|------|---|---|-----|---|-----|---|----|---|
| Yrel | +1 | 0 | 0 | 0 | 0 | 0 | -1 | 0 |
| ms | 0 | 2 | 4 | 6 | 8 | 10 | 12 | 14 |

A

| Pup. pos. | 0 | 2 | 4 | 6 | 8 | 10 | 12 | 14 | 16 | 18 | 20 | 22 |
|-----------|---|---|---|---|---|----|----|----|----|----|----|----|
| Xpup abs | 0 | +1 | +1 | 0 | 0 | +1 | +1 | 0 | -1 | 0 | +1 | 0 |
| Ypup abs | 0 | 0 | +1 | +1 | 0 | -1 | +2 | +1 | +1 | 0 | +2 | +1 |

B          C

EP 1 923 026 B1

|     | -2  | -1  | 0   | +1  | +2  |
|-----|-----|-----|-----|-----|-----|
| +2  | 0   | 0   | 0   | 0   | 0   |
| +1  | 0   | 0   | 2   | 0   | 0   |
| 0   | 0   | 0   | 2   | 0   | 0   |
| -1  | 0   | 1   | 2   | 1   | 0   |
| -2  | 0   | 0   | 0   | 0   | 0   |

FIG 5A

EP 1 923 026 B1

| Xpuls abs | 0 | 0 | -1 | 0 | 0 | +1 | 0 | 0 |
|---|---|---|---|---|---|---|---|---|
| Ypuls abs | +1 | +1 | -1 | 0 | -1 | -1 | -1 | 0 |
| ms | 0 | 2 | 4 | 6 | 8 | 10 | 12 | 14 |

FIG 5B

EP 1 923 026 B1

|     | -2  | -1  | 0   | +1  | +2  |
| --- | --- | --- | --- | --- | --- |
| +2  | 0   | 0   | 0   | 0   | 0   |
| +1  | 0   | 0   | 2   | 0   | 0   |
| 0   | 0   | 1   | 4   | 1   | 0   |
| -1  | 0   | 1   | 2   | 1   | 0   |
| -2  | 0   | 0   | 0   | 0   | 0   |

FIG 6A

| Xrel | 0 | 0 | -1 | 0 | 0 | +1 | 0 | 0 |
|---|---|---|---|---|---|---|---|---|
| Yrel | +1 | +1 | -1 | 0 | -1 | -1 | -1 | 0 |
| ms | 0 | 2 | 4 | 6 | 8 | 10 | 12 | 14 |

| Xrel | -1 | 0 | +1 | 0 |
|---|---|---|---|---|
| Yrel | 0 | 0 | 0 | 0 |
| ms | 24 | 26 | 28 | 30 |

FIG 6B

EP 1 923 026 B1

EP 1 923 026 B1

|     | -2  | -1  | 0   | +1  | +2  |
|-----|-----|-----|-----|-----|-----|
| +2  | 0   | 0   | 0   | 0   | 0   |
| +1  | 0   | 0   | 2   | 0   | 0   |
| 0   | 0   | 2   | 5   | 2   | 0   |
| -1  | 0   | 1   | 2   | 1   | 0   |
| -2  | 0   | 0   | 0   | 0   | 0   |

FIG 7A

| Xrel | 0 | 0 | -1 | 0 | 0 | +1 | 0 | 0 |
|------|----|----|----|----|----|----|----|----|
| Yrel | +1 | +1 | -1 | 0 | -1 | -1 | -1 | 0 |
| ms | 0 | 2 | 4 | 6 | 8 | 10 | 12 | 14 |

| Xrel | -1 | 0 | +1 | 0 | -1 | 0 | +1 |
|------|----|----|----|----|----|----|----|
| Yrel | 0 | 0 | 0 | 0 | 0 | 0 | 0 |
| ms | 24 | 26 | 28 | 30 | 32 | 34 | 36 |

FIG 7B

EP 1 923 026 B1

| +2 | +1 | 0 | -1 | -2 |    |
|----|----|---|----|----|----|
| 0  | 0  | 0 | 0  | 0  | +2 |
| 0  | 1  | 3 | 1  | 0  | +1 |
| 0  | 3  | 6 | 3  | 0  | 0  |
| 0  | 1  | 3 | 1  | 0  | -1 |
| 0  | 0  | 0 | 0  | 0  | -2 |

FIG 8A

| Xrel | 0 | 0 | -1 | 0 | 0 | +1 | 0 | 0 |
|------|---|---|----|---|---|----|---|---|
| Yrel | +1 | +1 | -1 | 0 | -1 | -1 | -1 | 0 |
| ms | 0 | 2 | 4 | 6 | 8 | 10 | 12 | 14 |

| Xrel | -1 | 0 | +1 | 0 | -1 | 0 | +1 | 0 | ... |
|------|----|---|----|---|----|---|----|---|-----|
| Yrel | 0 | 0 | 0 | 0 | 0 | 0 | 0 | +1 | ... |
| ms | 24 | 26 | 28 | 30 | 32 | 34 | 36 | 38 | 40... |

# FIG 8B

EP 1 923 026 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0189438 A